Europäisches Patentamt

European Patent Office

Office européen des brevets

(19)

(11) Veröffentlichungsnummer: **0 264 750**

**A2**

(12) # EUROPÄISCHE PATENTANMELDUNG

(21) Anmeldenummer: **87114814.4**

(22) Anmeldetag: **10.10.87**

(51) Int. Cl.⁴: **C07K 7/10** , **A61K 37/02** , **A23K 1/16**

(30) Priorität: **23.10.86 US 922572**

(43) Veröffentlichungstag der Anmeldung:
**27.04.88 Patentblatt 88/17**

(84) Benannte Vertragsstaaten:
**AT BE CH DE FR GB IT LI NL**

(71) Anmelder: **F. HOFFMANN-LA ROCHE & CO.**
**Aktiengesellschaft**

**CH-4002 Basel(CH)**

(72) Erfinder: **Felix, Arthur M.**
**257 Central Avenue**
**West Caldwell, N.J. 07006(US)**
Erfinder: **Heimer, Edgar P.**
**87 Edison Terrace**
**Sparta, N.J. 07871(US)**
Erfinder: **Mowles, Thomas F.**
**266 Change Bridge**
**Pine Brook, N.J. 07058(US)**

(74) Vertreter: **Mezger, Wolfgang, Dr. et al**
**Grenzacherstrasse 124 Postfach 3255**
**CH-4002 Basel(CH)**

(54) **Wachstumshormon-Releasingfaktor-Analoga.**

(57) Die Erfindung betrifft GRF-Analoga folgender Formel

X -R¹-R²-Asp-Ala-Ile-Phe-Thr-Asn-Ser-Tyr-Arg-R¹²-Val-Leu-R¹⁵-Gln-Leu-Ser-Ala-Arg-R²¹-Leu-Leu-Gln-Asp-Ile-R²⁷-R²⁸-Arg-Gln-Gln-Gly-Glu-Ser-Asn-Gln-Glu-Arg-Gly-Ala-Arg-Ala-Arg-Leu-Y

worin

$R^1$ für Tyr, D-Tyr, desNH₂-Tyr, Acyl-Tyr, His oder $C^\alpha$-Methyl-Tyr;
$R^2$ für Ala, N-Methyl-D-Ala oder D-Ala;
$R^{12}$ für Lys, Ala, Leu, Val oder Ile;
$R^{15}$ für Ala, Leu, Val, Ile, Nle, Nval, β-Ala oder α-Aib;
$R^{21}$ für Lys, Ala, Leu, Val oder Ile ;
$R^{27}$ für Met, Leu, Nle oder Ile;
$R^{28}$ für Asn oder Ser;
X für Wasserstoff oder -CO-A;
Y für -OE oder -NGL;

A für Wasserstoff, C₁₋₄-Alkyl oder Halogen-C₁₋₄-alkyl;
E für Wasserstoff oder C₁₋₅-Alkyl;
G und L unabhängig voneinander für beide entweder Wasserstoff, C₁₋₇-Alkyl, C₂₋₄-Alkenyl oder Halo-C₁₋₄-alkyl stehen;
mit der Einschränkung, dass, wenn E oder G und L beide Wasserstoff darstellen, wenigstens einer der Substituenten X, R¹, R¹², R²¹ und R²⁸ einen der unterstrichenen Reste darstellt, um eine bis 15 Aminosäuren C-terminal verkürzte Fragmente davon, sowie pharmazeutisch verträgliche Säure-oder Basenadditionssalze dieser Verbindungen.

Die GRF-Analoga und ihre pharmazeutischen Verbindungen sind zur Behandlung wachstumshormonbedingter Störungen beim Menschen und zur Anwendung bei Tieren geeignet.

## Wachstumshormon-Releasingfaktor-Analoga

Die vorliegende Erfindung bezieht sich auf Releasingfaktor-Analoga des menschlichen Wachstumshormons und Fragmente davon sowie auf pharmazeutische Zusammensetzungen, die solche Analoga oder Fragmente davon enthalten. Die erfindungsgemässen pharmazeutischen Zusammensetzungen können zur Behandlung verschiedener, in Tier wie Mensch auftretender, Wachstumshormon bedingter Krankheiten verwendet werden.

Der Releasingfaktor des Wachstumshormons (GRF) ist aus einem Tumor menschlicher Inselzellen isoliert und seine Struktur von Guillemin et al. am Salk Institute (Science 218 :585 [1982]) aufgeklärt worden: Jahrzehntelang entzog sich GRF der Isolierung und Charakterisierung, da das Polypeptid nur in sehr geringen Mengen im Gewebe vorhanden ist. Es stellte sich heraus, dass der Releasingfaktor (hGRF) des Wachstumshormons des menschlichen Hypothalamus über die gleiche Struktur wie der aus den Inselzellen des Tumors isolierte GRF verfügt (Bohlen et al., Biochem. Biophys. Res. Commun. 114:930 [1983]).

Rivier et al. (Nature 300:276 [1982]) haben die Struktur von GRF(1-44) und GRF(1-40) beschrieben und haben gezeigt, dass GRF in spezifischer Weise die Freisetzung des Wachstumshormons beeinflusst. Diese beiden Formen des GRF verfügen über denselben Amino-Terminus, unterscheiden sich aber hinsichtlich der Länge und des C-Terminus, der im Fall von GRF(1-44) amidiert ist.

Rivier et al. (supra) haben gezeigt, dass sich die biologische Aktivität von GRF im aminoterminalen Teil des Moleküls befindet. Eine uneingeschränkte Wirksamkeit konnte mit GRF(1-29)-$NH_2$ in vitro gezeigt werden.

Lance et al. (Biochem. Biophys. Res. Commun. 119:265 [1984]) haben gezeigt, dass an den Positionen 1, 2 und 3 substituiertes GRF(1-29)-$HN_2$ zu vermehrter Freisetzung von Wachstumshormon (GH), sowohl in Schweinen wie in Ratten, führt. Das U.S. Patent Nr. 4,518,586 offenbart verschiedene, synthetische GRF Peptide, wobei sich eines durch eine Substitution des Glycins in Position 15 durch D-Ala auszeichnet. Auch die U.S. Patente Nr. 4,528,190 und 4,529,595 offenbaren verschiedene GRF-Analoga einschliesslich solcher mit Substitutionen von D-Ala in Position 15 und von Asn in Position 28. Die unter Nr. 177,819 publizierte europäische Patentanmeldung offenbart GRF-Analoga, in denen Glycin in Position 15 durch Alanin, Leucin, Valin, Isoleucin, Norleucin, Norvalin, β-Alanin oder α-Aminoisobuttersäure ersetzt ist.

Man glaubt, dass das Wachstum von Tieren durch eine Kaskade regulatorischer Moleküle gesteuert wird. Der Hypothalamus synthetisiert GRF, welcher die Freisetzung des Wachstumshormons aus der Hypophyse induziert. Es stellte sich heraus, dass geringe Mengen von GRF die Freisetzung einer beträchtlichen Menge von Wachstumshormon aus der Hypophyse ins Blut verursacht. So kommt GRF eine grosse therapeutische Bedeutung in solchen Fällen, in denen die Gabe von Wachstumshormon angezeigt ist, zu. GRF kann beispielsweise in der Behandlung von hypophysärem Zwergwuchs, einem durch Störung der Synthese des Wachstumshormons bedingten Diabetes, zur Förderung des Heilungsprozesses von Wunden und Knochenfrakturen, zur Behandlung von Verbrennungen und zur Verzögerung des Alterungsprozesses eingesetzt werden. In ähnlicher Weise kann GRF in der Landwirtschaft eingesetzt werden; beispielsweise zur Steigerung der Fleischproduktion bei Geflügel, Schweinen, Rindern oder anderem Getier, was eine frühzeitigere Vermarktung ermöglicht, oder die Produktion grösserer Tiere bei gleichbleibender Aufzuchtszeit oder die Steigerung des Fleischanteils gegenüber dem Fettanteil. GRF kann auch die Produktion von Milch und Eiern stimulieren.

Die vorliegende Erfindung bezieht sich auf 29-44 Aminosäuren lange neue Peptide folgender Formel

X -R¹-R²-Asp-Ala-Ile-Phe-Thr-Asn-Ser-Tyr-Arg-R¹²-Val-Leu-R¹⁵-Gln-Leu-Ser-Ala-Arg-R²¹-Leu-Leu-Gln-Asp-Ile-R²⁷-R²⁸-Arg-Gln-Gln-Gly-Glu-Ser-Asn-Gln-Glu-Arg-Gly-Ala-Arg-Ala-Arg-Leu-Y     I

worin

| | |
|---|---|
| R¹ | für Tyr, D-Tyr, desNH₂-Tyr, Acyl-Tyr, His oder Cᵅ-Methyl-Tyr; |
| R² | für Ala, N-Methyl-D-Ala oder D-Ala; |
| R¹² | für Lys, Ala Leu, Val oder Ile; |
| R¹⁵ | für Ala, Leu, Val, Ile, Nle, Nval, β-Ala oder α-Aib; |
| R²¹ | für Lys, Ala, Leu, Val oder Ile ; |
| R²⁷ | für Met, Leu, Nle oder Ile; |
| R²⁸ | für Asn oder Ser; |
| X | für Wasserstoff oder -CO-A; |
| Y | für -OE oder -NGL; |
| A | für Wasserstoff, C ₁₋₄-Alkyl oder Halo-C₁₋₄-alkyl; |
| E | für Wasserstoff oder C ₁₋₅-Alkyl; |

G und L unabhängig voneinander beide für Wasserstoff, C₁₋₇-Alkyl, C₂₋₄-Alkenyl oder Halo-C₁₋₄-alkyl

stehen;

mit der Einschränkung, dass, wenn E oder G und L beide Wasserstoff darstellen, wenigstens einer der Substituenten X, $R^1$, $R^{12}$, $R^{21}$ und $R^{28}$ einen der unterstrichenen Reste darstellt. Die vorliegende Erfindung bezieht sich auch auf um eine bis 15 Aminosäuren C-terminal verkürzte Fragmente gemäss obiger Formel. Die Erfindung umfasst auch pharmazeutisch verträgliche Säure-oder Basenadditionssalze obiger Verbindungen.

Bevorzugte Peptide gemäss Formel I enthalten 29, 30, 32 oder 40, insbesondere 29, 30 oder 32 Aminosäuren (gezählt vom N-terminalen Ende).

In einer anderen bevorzugten Ausführungsform steht X für Wasserstoff, $R^1$ für Tyr oder desNH$_2$-Tyr, $R^2$ für Ala oder D-Ala, $R^{12}$ für Lys oder Ala, $R^{15}$ für Ala, $R^{21}$ für Lys oder ·Ala, $R^{27}$ für Leu oder Nle und das Peptid hat eine Länge von 32 Aminosäuren (gezählt vom N-Terminus) und einen freien C-Terminus.

Besonders bevorzugte erfindungsgemässe Verbindungen sind
[Ala$^{15}$, Leu$^{27}$,Asn$^{28}$]-GRF(1-32)-OH und
[Ala$^{15}$, Leu$^{27}$,Asn$^{28}$]-GRF(1-40)-OH.

Die Erfindung bezieht sich ferner auf pharmazeutische Zusammensetzungen, die die neuen Peptide gemäss Formel I in Verbindung mit einem pharmazeutisch sinnvollen, flüssigen oder festen Trägermaterial enthalten, sowie auf die Verwendung der neuen erfindungsgemässen Peptide gemäss Formel I zur Förderung des Wachstums von Mensch wie Warm-und Kaltblütlern, wie z.B. Rindern, Schweinen, Geflügel und Fischen. Die Peptide gemäss Formel I sind besonders nützlich zur Behandlung von an Wachstumshormonmangel leidenden Individuen, besonders während der Entwicklungszeit, wie auch zur Behandlung von Kindern mit Zwergwuchs.

In obiger Formel wurde die übliche Aminosäurenomenklatur verwendet. Nle bedeutet Norleucin, Nval bedeutet Norvalin, α-Aib bezieht sich auf α-Aminoisobuttersäure und β-Ala bezieht sich auf β-Alanin. Bei Aminosäureisomeren ist, falls nicht anders angegeben, die L-Form gemeint.

Die Suffixe "-OH" und "-NH$_2$" bezeichnen in der vorliegenden Beschreibung die freie Säure bzw. die amidierte Form der erfindungsgemässen Polypeptide. Wird kein Suffix verwendet, sind beide Formen gemeint. GRF-Analoga werden durch Vorsetzen der entsprechenden, substituierten Aminosäure in Klammern bezeichnet: "(Ala$^{15}$)-GRF" steht beispielsweise für ein Polypeptidn in dessen Aminosäuresequenz Glyzin in Position 15 durch einen Alaninrest ersetzt wurde. Hinter GRF gesetzte Zahlen in Klammern bezeichnen die Anzahl der Aminosäurereste der Fragmente. Beispielsweise steht GRF(1-29) für ein Fragment, das die ersten 29 Aminosäurereste der vollen Sequenz umfasst.

Die Ausdrücke "C$_{1-4}$-Alkyl", "C$_{1-5}$-Alkyl" und "C$_{1-7}$-Alkyl" beziehen sich auf gerad-bzw. verzweigtkettige Alkylgruppen mit 1-4, 1-5 und 1-7 Kohlenstoffatomen, wie beispielsweise Methyl, Ethyl, n-Propyl, Isopropyl, n-Butyl, Isobutyl und tert.Butyl. Der Ausdruck "Halo-C$_{1-4}$-alkyl" bezieht sich auf eine Alkylgruppe mit 1-4 Kohlenstoffatomen, die durch Chlor, Brom, Fluor oder Jod, wie beispielsweise bei -CH$_2$Cl, -CHCl$_2$, -CH$_2$F, -CF$_3$, -CH$_2$CH$_2$Cl, -CH$_2$CCl$_3$, substituiert wurde. Der Ausdruck "C$_{2-4}$-Alkenyl" bezieht sich auf Gruppen wie -CH-CH$_2$, -CH$_2$CH = CH$_2$, -CH = CHCH$_2$, CH$_2$CH$_2$CH = CH$_2$.

Pharmazeutisch sinnvolle Salze der erfindungsgemässen GRF-Analoga sind beispielsweise Metallkomplexe mit Zink oder Eisen. Beispiele von Säureadditionssalzen sind Hydrochloride, Hydrobromide, Sulfate, Phosphate, Maleate, Ace tate, Citrate, Benzoate, Succinate, Maleate, Ascorbate, Tartrate und ähnliche mehr.

Repräsentative erfindungsgemässe Peptide gemäss Formel I sind in Tabelle 1 gezeigt.

Tabelle 1

| X | R^1 | R^2 | R^12 | R^15 | R^21 | R^27 | R^28 | Y |
|---|---|---|---|---|---|---|---|---|
| H | Tyr | Ala | Lys | Ala | Lys | Leu | Asn | $-NH_2$ |
| $-COCH_3$ | D-Tyr | Ala | Leu | Ala | Ala | Met | Asn | $-OH$ |
| $-COC_2H_5$ | D-Tyr | D-Ala | Ala | Leu | Leu | Leu | Asn | $-OCH_3$ |
| $-COCH_3$ | Tyr | N-Methyl-Ala | Leu | Val | Val | Met | Asn | $-OC_2H_5$ |
| H | D-Tyr | Ala | Val | Ala | Ile | Ile | Asn | $-OH$ |
| $-COC_3H_7$ | Tyr | N-Methyl-Ala | Lys | Ile | Ala | Nle | Asn | $-NHCH_3$ |
| H | desNH$_2$-Tyr | Ala | Ile | Nle | Lys | Met | Asn | $-NHC_2H_5$ |
| $-COCH_3$ | Tyr | D-Ala | Val | Ile | Ala | Met | Asn | $-N(CH_3)_2$ |
| H | desNH$_2$-Tyr | Ala | Leu | Ala | Leu | Leu | Asn | $-N(C_2H_5)_2$ |
| H | His | Ala | Ala | Val | Ile | Leu | Asn | $-NHCH-CH_2$ |
| H | Tyr | N-Methyl-Ala | Lys | Nle | Val | Met | Asn | $-NHCH=CH_2$ |
| $-COCH_3$ | D-Tyr | Ala | Leu | Nval | Ala | Ile | Asn | $-NH_2$ |
| H | His | Ala | Val | Ala | Leu | Nle | Asn | $-OH$ |
| H | Tyr | D-Ala | Ala | Ala | Leu | Met | Asn | $-NHCCl_3$ |

Die erfindungsgemässen Polypeptide können mittels bekannter Techniken wie Fest-bzw. Flüssigphasenpeptidsynthese oder via rekombinante DNA-Technologie hergestellt werden. Rekombinante DNA-Techniken könnten zur Herstellung eines Teils des Peptids, nämlich eines solchen, der aus nur natürlich vorkommenden Aminosäuren besteht, verwendet werden. Dieser Teil des Moleküls könnte dann mit einem zweiten synthetisch hergestellten, der Aminosäuren enthält, die nicht vom genetischen Kode kodiert werden, verbunden werden. Festphasensynthese, wie von Merrifield, J. Am. Chem. Soc. 85 :2149 (1963) beschrieben, wie auch andere gleichwertige, bekannte chemische Synthesemethoden können zur Herstellung der erfindungsgemässen Peptide verwendet werden.

Die Festphasensynthese beginnt mit der C-terminalen Aminosäure des zu synthetisierenden Peptids durch Kopplung der geschützten Aminosäure an ein passendes Trägermaterial. Das Ausgangsmaterial kann durch Bindung der aminogeschützten, C-terminalen Aminosäure über eine Benzylesterbrücke an chlormethyliertes oder hydroxymethyliertes Trägermaterial oder über eine Amidbindung an Benzhydrylamin (BHA)-oder Methylbenzhydrylamin (MBHA)-Trägermaterial hergestellt werden. Diese Trägermaterialien sind käuflich erhältlich und ihre Verwendung ist wohlbekannt.

Die neuen erfindungsgemässen GRF-Analoga mit freiem C-Terminus können durch Festphasensynthese mittels eines Benzylesterträgermaterials hergestellt werden. Die Polypeptide können bis zur Homogenität durch präparative Hochleistungsflüssigkeitschromatographie (HPLC) gereinigt werden. Die Reinheit dieser Peptide kann mittels analytischer HPLC, isoelektrischer Fokussierung und Hochspannungsdünnschichtelektrophorese gezeigt werden. Mittels Aminosäureanalyse kann die erwartete Aminosäurezusammensetzung bestätigt werden.

Die entsprechenden Amide können durch Festphasenpeptidsynthese an BHA-oder MBHA-Trägermaterialien hergestellt werden. Dem Fachmann ist klar, dass bei Verwendung von BHA-oder MBHA-Trägermaterialien Umsetzung mit wasserfreier HF, zur Entfernung des Polypeptids von der Festphase, ein Polypeptid mit amidiertem C-Terminus entsteht.

Die N-α-Aminogruppe der C-terminalen Aminosäure, wie beispielsweise bei Gly, ist durch eine t-Butyloxycarbonyl-(Boc)-Gruppe geschützt. Boc-Gly-OH wird dann mittels Dicyclohexylcarbodiimid (DCC) bei 25°C unter zweistündigem Rühren an das Trägermaterial gekoppelt. Danach wird mittels Trifluoressigsäure (TFA) in Methylenchlorid oder mittels reiner TFA die α-Aminoschutzgruppe entfernt. Diese Reaktion wird zwischen 0°C und Raumtemperatur durchgeführt.

Allgemeine Methoden zum Schützen von Aminosäuren und Entfernung der Schutzgruppen von den Aminosäuren, die in der vorliegenden Erfindung verwendet werden können, sind beispielsweise in "The Peptides", Vol. 2 (E. Gross und J. Meienhofer, Herausg.), Academic Press, New York, 1-284 (1979), beschrieben.

Nach Entfernen der α-Aminoschutzgruppe von der an das Trägermaterial gebundenen Aminosäure werden die weiteren Aminosäuren - schrittweise in der gewünschten Reihenfolge in Boc-geschützter Form angekoppelt. Andererseits können auch einzelne Aminosäuren ausserhalb der Festphasensynthesemaschine zu einem Peptid synthetisiert und anschliessend in die Maschine gegeben werden. Die Auswahl der entsprechenden Kopplungsreagenzien stellt für den Fachmann kein Problem dar. Besonders geeignet ist DCC.

Jede geschützte Aminosäure oder jedes geschützte Peptid wird im Ueberschuss in die Festphasenmaschine gegeben. Die Kopplung kann in Dimethylformamid (DMF) oder Methylenchlorid (CH2Cl2) oder in Mischungen davon durchgeführt werden. In Fällen unvollständiger Kopplung wird vor Entfernen der N-α-Aminoschutzgruppe zur Kopplung der nächsten Aminosäure der vorhergehende Kopplungsschritt nochmals wiederholt. Die Ausbeute jedes Kopplungsschrittes kann, vorzugsweise mittels der Ninhydrinreaktion, verfolgt werden. Die Kopplungsreaktionen können in einer Peptidsynthesemaschine, Modell Vega 250, durchgeführt werden.

Die Abspaltung des fertigen Peptids vom Trägermaterial kann mittels in der Peptidchemie bekannter Methoden durchgeführt werden. Beispielsweise durch einstündige Umsetzung mit Fluorwasserstoff in Gegenwart von p-Cresol und Dimethylsulfid bei 0°C und, falls gewünscht, anschliessende zweistündige Umsetzung mit Fluorwasserstoff in Gegenwart von p-Cresol bei 0°C.

Die Reinigung der erfindungsgemässen Polypeptide kann mittels in der Peptidchemie bekannter Methoden durchgeführt werden. Wie bereits erwähnt, ist Reinigung mittels präparativer HPLC möglich, jedoch können auch andere chromatographische Verfahren wie Gelfiltration, Ionenaustaustaucher-, Verteilungsgegenstrom-oder Verteilungschromatographie verwendet werden.

Die erfindungsgemässen Polypeptide verfügen über eine Aktivität, die zur Freisetzung von Wachstumshormon führt. Erfindungsgemässe pharmazeutische Zusammensetzungen sind Dispersionen von 29-44 Aminosäuren langen GRF-Analoga oder deren nicht-toxischen Salzen in einer pharmazeutisch verträglichen flüssigen oder festen Trägermaterial. Die erfindungsgemässen Verbindungen und pharmazeutischen Zusammensetzungen können in der Humanmedizin zur Steigerung der GH-Synthese verwendet werden. Simulierung der GH-Sekretion durch die erfindungsgemässen Peptide ist bei Patienten mit vollständigem oder relativem GH-Mangel, der durch Unterproduktion von endogenem GRF verursacht wird, in der Behandlung von hypophysärem Zwergwuchs und bei durch abnormale GH-Synthese bedingtem Diabetes von Interesse. Ferner ist es auch möglich, dass eine erhöhte GH-Sekretion, die zu stärkerem Wachstum führt, bei Mensch wie Tier mit normalen GH-Werten erhalten werden kann. Darüberhinaus sollte die Anwendung der neuen Verbindungen den Fettanteil verändern und andere GH-abhängige metabolische, immunologische und Entwicklungsprozesse beeinflussen. Beispielsweise könnten die erfindungsgemässen Peptide zur Stimmulation anabolischer Prozesse im Menschen, z.B. bei Behandlung von Verbrennungen, verwendet werden. Im weiteren könnten sie in der Landwirtschaft gehaltenen Warmblütlern wie Hühnern, Truthähnen, Schweinen, Ziegen, Rindern und Schafen zur Stimmulation des Wachstums oder einer verstärkten Futterausnützung bei zu Fleisch-, Milch-oder Eierproduktion gehaltenen Tiere verabreicht werden. Sie könnten auch in Aquakulturen zur Aufzucht von Fischen und anderen Kaltblütlern, wie beispielsweise Meeresschildkröten und Amphibien, zur Steigerung des Wachstums und der Erhöhung des Proteinanteils gegenüber dem Fettanteil verwendet werden.

Zur Anwendung am Menschen sollten die erfindungsgemässen Peptide eine Reinheit von wenigstens 93%, vorzugsweise über 98% haben. Der Ausdruck Reinheit bezieht sich auf den Anteil des gewünschten Peptids an der Menge aller vorhandenen Peptide und Peptidfragmente. Zur Steigerung des Wachstums und zur Verminderung des Fettgehalts von Tieren ist eine Reinheit von 3% bzw. von wenigstens 0.01% akzeptabel.

Die biologische Aktivität der erfindungsgemässen Verbindungen wurde mit der von synthetischem $GRF(1-44)-NH_2$, das vergleichbar ist mit dem natürlichen GRF $(1-44)-NH_2$, verglichen. Die biologische Aktivität des synthetischen $GRF(1-44)-NH_2$ entsprach dem natürlichen $GRF(1-44)-NH_2$, das aus einem menschlichen Pankreastumor eines an Akromegalie leidenden Individuums (Salk Institute-Standard $hGRF-NH_2(NL-A-10)$) isoliert worden war. Der Nachweis der biologischen Aktivität, der auf der Fähigkeit beruht, die Synthese von GH in Rattenhypophysenzellen in Gewebekultur zu stimulieren, wurde wie in Beispiel 2 beschrieben durchgeführt.

Geeignete Dosierungen der erfindungsgemässen Polypeptide werden von der behandelten Person und den jeweiligen Umständen abhängen. Der Fachmann wird die geeignete Dosis auf der Grundlage bekannter Wachstumshormonspiegel normalen Wachstums und der Kenntnis der Freisetzungsaktivität der Polypeptide bestimmen können. Wie bekannt, wird die Behandlung wachstumsbedingter Störungen eine individuelle Dosierung in Abhängigkeit des Insuffizienzgrades der Wachstumshormonproduktion erfordern.

Im allgemeinen kann eine Dosis von 0.04 µg/kg/Tag bis zu 1,0 µg/kg/Tag bezogen auf das Körpergewicht der zu behandelnden Person zur Stimulation der Freisetzung des Wachstumshormons verwendet werden. Die Dosis zur Stimulation des Wachstums von Vieh wird wesentlich höher sein als die am Menschen zur Wiederherstellung normalen Wachstums bei Wachstumshormonmangel wie im Fall von hypophysärem Zwergwuchs, verwendete. In der Viehzucht wird im allgemeinen eine Dosis von 0,4 µ/kg/Tag bis 10 µg/kg/Tag subkutan zur Stimulierung der Freisetzung von Hypophysenwachstumhormon appliziert.

Die zur Human-bzw. Veterinärmedizin geeigneten pharmazeutischen Zusammensetzungen können gemäss konventionellen pharmazeutischen Formulierungstechniken hergestellt werden. Zur oralen, intravenösen, subkutanen, intramuskulären, intraperitonealen, intranasalen Applikation geeignete Zusammensetzungen können hergestellt werden. Eine geeignete Dosis zur pharmazeutischen Verwendung besteht aus 0.01 bis 0,5 mg der erfindungsgemässen Verbindung, die zur Rekonstitution mit sterilem Wasser oder Kochsalzlösung lyophilisiert werden kann. Die Zusammensetzung sollte einem pH-Wert unterhalb von 8,0 gehalten werden, um die Stabilität des Peptids zu gewährleisten. Serumalbumin der zu behandelnden Spezies (beispielsweise humanes Serumalbumin im Fall von Mensch, Rinderserumalbumin im Fall von Rind, usw.) kann zusammen mit anderen bekannten pharmazeutischen Adjuvantien verwendet werden.

Die Erfindung bezieht sich auch auf Methoden zur Behandlung wachstumsbedingter Störungen, die durch eine unzureichende Synthese von Wachstumshormon charakterisiert sind und zwar durch Gabe einer zur Stimulation der Synthese des Wachstumshormons ausreichenden Menge, um einen Wachstumshormonspiegel zu erreichen, der zu normalem Wachstum führt.

Die normalen Serumspiegel von Wachstumshormon sind individuell und während des Tagesverlaufs stark unterschiedlich. Bei Erwachsenen können die normalen Serumspiegel von Wachstumshormon 0-10 Nanogramm/ml betragen. Bei Kindern können die normalen Serumspiegel von Wachstumshormon 0-20 Nanogramm/ml betragen.

Zur wirkungsvollen Behandlung von hypohysärem Zwergwuchs mit den beschriebenen GRF-Analoga wird die Behandlung während der Periode normalen Wachstums durchgeführt. Bei weiblichen Individuen reicht diese Periode nicht wesentlich über das Einsetzen der ersten Menstruation. So sollte die Behandlung weiblicher Individuen im Alter von 12-16 Jahren, individuell abgestimmt, beendet werden. Bei männlichen Individuen kann die Stimulation des Wachstums beträchtlich länger, über die Pubertät hinaus, fortgesetzt werden. So kann die wirkungsvolle Behandlung von männlichen Individuen normalerweise bis zum Alter von 18-19 Jahren und in einigen Ausnahmefällen bis zum 25. Altersjahr durchgeführt werden.

Schliesslich wird auch eine Methode zur Steigerung des Wachstums von Tieren mittels Applikation einer Menge an GRF-Analoga, die ausreicht, die Synthese von Wachstumshormon bis zu einem Spiegel zu steigern, der über dem normalen Wachstum liegt, zur Verfügung gestellt.

## Beispiele

In den folgenden Beispielen wurden, falls nicht ausdrücklich anders vermerkt, optisch aktive, geschützte Aminosäuren, in der L-Konfiguration verwendet. Die geschützten Aminosäuren wurden mittels Dünnschichtchromatographie auf Silicagelplatten Typ G, die mit Chlor-TDM entwickelt wurden, untersucht. Schmelzpunkte wurden mittels eines Thomas-Hoover-Apparates bestimmt und sind unkorrigiert angegeben. Die optischen Drehungen wurden in einer geschützten 1 dm-Zelle eines Perkin-Elmer-Polarimeters Modell 141 gemessen und auf die üblichen Werte umgerechnet. Die Aminosäurezusammensetzung wurde mittels eines Aminosäureanalysators von Waters durchgeführt.

Die folgenden Abkürzungen von verschiedenen Schutzgruppen und Reagenzien werden in den Beispielen verwendet:

BOC = t-Butyloxycarbonyl
Z = Benzyloxycarbonyl
2-ClZ = 2-Chlorbenzyloxycarbonyl
Bzl = Benzyl
2,6-Cl$_2$-Bzl = 2,6-Dichlorbenzyl
Tos = p-Toluolsulfonyl
DCC = Dicyclohexylcarbodiimid
BHA = Benzhydrylamin

PAM = Phenylacetamidomethyl
DMF = Dimethylformamid
DTE = Dithioethan
TFA = Trifluoressigsäure
CH$_2$Cl$_2$ = Methylenchlorid
TGA = Thioglycolsäure

Die erfindungsgemässen GRF-Analoga wurden durch aufeinanderfolgende Kopplung der Aminosäuren mittels einer käuflich erhältlichen Festphasenpeptidsynthesemaschine (Vega 250 Peptide Synthesizer) hergestellt, N-$\alpha$-Boc-Aminosäuren wurden zur Synthese verwendet.

Trifunktionelle Aminosäuren wurden in Form der folgenden geschützten Aminosäuren eingesetzt: N-$\alpha$-Boc-Lys(2-ClZ), N-$\alpha$-Boc-Asp(OBzl), N-$\alpha$-Boc-Glu(OBzl), N-$\alpha$-Boc-Ser(Bzl), N-$\alpha$-Boc-Thr(Bzl), N-$\alpha$-Boc-Tyr(2,6-Cl$_2$-Bzl) und N-$\alpha$-Boc-Arg(Tos).

Die folgenden Beispiele sollen die Erfindung veranschaulichen ohne ihren Umfang zu beschränken. Falls nicht anders erwähnt, sind Teil- und Prozentangaben auf das jeweilige Gewicht bezogen und die Temperaturen in °C angegeben.

## Beispiel 1

Herstellung von [Ala$^{15}$, Leu$^{27}$, Asn$^{28}$]-GRF(1-32)-OH.

Boc-Gly-PAM-Trägermaterial (Vega Biochemicals, 8 g, 0,41 mM/g) wurde in das Reaktionsgefäss einer Vega 296 Peptidsynthesemaschine gepackt. Die 5 Zyklen der Festphasenpeptidsynthese nach dem DCC-Verfahren führten zu Boc-Leu$^{27}$-Asn$^{28}$-Arg(Tos)$^{29}$-Gln$^{30}$-Gln$^{31}$-Gly$^{32}$-PAM-Trägermaterial (9,26 g). Das PAM-Trägermaterial (0,7 g, 0,21 mmol) wurde in das Reaktionsgefäss einer Peptidsynthesemaschine Modell 430A von Applied Biosystems, gepackt. 26 Zyklen Festphasensynthese nach dem symmetrischen Anhydridverfahren führten zu [Ala$^{15}$,Leu$^{27}$,Asn$^{28}$]-GRF(1-32)-PAM-Trägermaterial (1,3 g), das 2 Stunden bei 0°C mit wasserfreiem Fluorwasserstoff (10% DTE enthaltend) behandelt wurde. HF wurde bei 0°C (Hochvakuum; CaO-Falle) abgezogen und die rohe Mischung aus Peptid und Trägermaterial mit EtOAc behandelt und mit TFA extrahiert. Das Lösungsmittel wurde abgezogen, der Rückstand mit wasserfreiem Ether behandelt und getrocknet.

Ein Teil (100 mg) des Rohmaterials (0,524 g) wurde in 20 ml 0,025%iger TFA/H$_2$O aufgelöst, zentrifugiert, gefiltert (0,45 $\mu$ Typ HA Miliporefilter) und auf zwei hintereinandergeschaltete 1 × 25 cm Synchropak RP-P Säulen gegeben. Die Säulen wurden dann mit einem linearen Gradienten von 25% CH$_3$CN (0.025% TFA)-H$_2$O bis 40% CH$_3$CN

(0,025% TFA)-H₂O in 120 Minuten (mit einer Flussrate von 2 ml/Minute) eluiert. Fraktionen wurden gesammelt (eine Minute/Fraktion) und Aliquote davon mittels HPLC analysiert. Das Produkt erschien in den Fraktionen 41 und 42, die vereint, eingeengt und lyophilisiert wurden, was reines [Ala¹⁵, Leu²⁷, Asn²⁸]-GRF(1-32)-OH mit einer Ausbeute von 3,2 mg ergab.

Das Produkt war nach analytischer HPLC homogen und ergab die erwartete Aminosäurezusammensetzung (Hydrolyse:6N HCl mit 1% TGA; 110°C; 72 Stunden): Asp. 4.10; Thr, 0.93; Ser, 2.13; Glu, 4.20; Gly, 1.00; Ala, 3.96; Val, 1.06; Ile, 1.89; Leu, 5.09; Tyr, 1.80; Phe, 0.94; Lys, 2.01; Arg, 2.88.

In analoger Weise wurde reines [Ala¹⁵,Leu²⁷,Asn²⁸]-GRF(1-40)-OH hergestellt.

Beispiel 2

Biologische Aktivität von [Ala¹⁵,Leu²⁷,Asn²⁸]-GRF(1-32)-OH.

Hypophysen von 30-40 männlichen Sprague-Dawley Ratten (175 g) wurden nach Köpfen in aseptischer Weise entfernt. Die Hypophysenvorderlappen wurden gesammelt und dreimal in sterilem 0,025 M Hepespuffer (pH 7,35) gewaschen. Bei 37°C wurde in 20-30 ml Hepespuffer (pH 7,35), der Kollagenase (4 mg/ml) und Dispase (Protease Grad II, 2 mg pro ml) enthielt, eine Dispersion davon hergestellt. Nach leichtem 100-110minütigem Schütteln und Suspension mittels Pasteurpipette wurden die Zellen durch Zentrifugation (150 × g, 4 Minuten) getrennt und in Hepespuffer (pH 7,35), der Neuraminidase (8 g/ml) und 200 g/ml des Dinatriumsalzes der Aethylendiamintetraessigsäure (EDTA) enthielt, 10 Minuten resuspendiert.

Die Zellen wurden zweimal mit dem zum Ausplattieren verwendeten Medium gewaschen und auf Platten mit Mehrfach-Vertiefungen (1,5 × 10⁵ Zellen pro ml) mittels des vorher definierten Mediums ausplattiert: F-12/DMEM/BGJ (6:3:1) (Gibco: 430-1700/430-1600/320-2591) mit 2 g BSA/1, 2,38 g Hepes/1,50 mg PSN Antibiotikamischung (Gibco Laboratories), 10 g/l Transferrin (Sigma T2252) mit 1,83 g Natriumhydrogencarbonat pro Liter (Baker 3506). Das Medium in jeder Vertiefung wurde entweder mit einer Probe der neuen GRF-Peptide oder natürlichem GRF(1-44)-NH₂ in Konzentrationen von 0.8 bis 200 fmol pro ml Medium angereichert. Kontrollen wurden ohne diese Anreicherungen durchgeführt. Zum Ausplattieren wurden dieses Medium, um eine schnelle Fixierung der Zellen zu erreichen, mit 2% fötalem Kälberserum verwendet.

Am vierten Tag wurden die Zellen zweimal mit dem definierten Medium, ohne fötales Kälberserum, gewaschen. Schliesslich wurden 900 μl des definierten Mediums und 100 μl des entsprechend behandelten Mediums in jede Vertiefung gegeben. Die Versuche wurden dreifach durchgeführt. Nach vierstündiger Inkubation wurde das Medium gesammelt und so verdünnt, dass ein Radioimmunassay (RIA) für Rattenwachstumshormon durchgeführt werden konnte.

Rattenwachstumshorm RIAs wurden mittels des anti-Murin GH-Immunserums von Sinha und einem Verfahren der National Pituitary Agency mittels Protein A zum Fällen des Antikörper-Antigenkomplexes durchgeführt. Die Ergebnisse sind in Tabelle 2 zusammengefasst.

Tabelle 2

Wirksamkeit von [Ala¹⁵,Leu²⁷, Asn²⁸]-GRF(1-32)-OH im Verhältnis zu GRF(1-44)-NH₂

| | |
|---|---|
| GRF(1-44)-NH₂ | 1.0 |
| [Ala¹⁵,Leu²⁷,Asn²⁸]-GRF(1-32)-OH | 2.2 |

Ansprüche

1. Ein Peptid der Formel

X -R¹-R²-Asp-Ala-Ile-Phe-Thr-Asn-Ser-Tyr-Arg-R¹²-Val-Leu-R¹⁵-Gln-Leu-Ser-Ala-Arg-R²¹-Leu-Leu-Gln-Asp-Ile-R²⁷-R²⁸-Arg-Gln-Gln-Gly-Glu-Ser-Asn-Gln-Glu-Arg-Gly-Ala-Arg-Ala-Arg-Leu-Y     I

worin

R¹     für Tyr, D-Tyr, desNH₂-Tyr, Acyl-Tyr, His oder Cᵅ-Methyl-Tyr ;
R²     für Ala, N-Methyl-D-Ala oder D-Ala;
R¹²     für Lys, Ala Leu, Val oder Ile;
R¹⁵     für Ala, Leu, Val, Ile, Nle, Nval, β-Ala oder α-Aib;
R²¹     für Lys, Ala, Leu, Val oder Ile;
R²⁷     für Met, Leu, Nle oder Ile;
R²⁸     für Asn oder Ser;
X     für Wasserstoff oder -CO-A;
Y     für -OE oder -NGL;
A     für Wasserstoff, C₁₋₄-Alkyl oder Halogen-C₁₋₄-alkyl;
E     für Wasserstoff oder C₁₋₅-Alkyl;
G und L unabhängig voneinander für beide entweder Wasserstoff, C₁₋₇-Alkyl, C₂₋₄-Alkenyl oder Halo-C₁₋₄-alkyl stehen;

mit der Einschränkung, dass, wenn E oder G und L beide Wasserstoff darstellen, wenigstens einer der Substituenten X, $R^1$, $R^{12}$, $R^{21}$ und $R^{28}$ einen der unterstrichenen Reste darstellt, um eine bis 15 Aminosäuren C-terminal verkürzte Fragmente davon,

sowie pharmazeutisch verträgliche Säure-oder Basenadditionssalze dieser Verbindungen.

2. Eine Verbindung gemäss Anspruch 1, worin $R^{28}$ Asn ist.

3. Eine Verbindung gemäss Anspruch 1 oder 2, worin $R^1$ desNH$_2$-Tyr oder Tyr ist.

4. Eine Verbindung gemäss einem der Ansprüche 1-3, worin $R^2$ Ala, N-Methyl-D-Ala oder D-Ala ist.

5. Eine Verbindung gemäss einem der Ansprüche 1-4, worin $R^{15}$ Ala ist.

6. Eine Verbindung gemäss einem der Ansprüche 1-5, die 29, 30, 32 oder 40 Aminosäuren enthält.

7. Eine Verbindung gemäss einem der Ansprüche 1-6, worin $R^{21}$ Ala oder Lys und X Wasserstoff ist.

8. Eine Verbindung gemäss einem der Ansprüche 1-7 worin $R^{12}$ Ala oder Lys und X Wasserstoff ist.

9. Eine Verbindung gemäss einem der Ansprüche 1-8, worin $R^{27}$ Leu ist.

10. Ein Peptid gemäss Anspruch 1, worin X Wasserstoff, $R^1$ Tyr oder desNH$_2$-Tyr, $R^2$ Ala oder D-Ala, $R^{12}$ Lys oder Ala, $R^{15}$ Ala, $R^{21}$ Lys oder Ala, $R^{27}$ Leu oder Nle und $R^{28}$ Asn oder Ser ist, wobei dieses Peptid 32 der N-terminalen Aminosäuren umfasst und über einen freien C-Terminus verfügt.

11. [Ala$^{15}$,Leu$^{27}$,Asn$^{28}$]-GRF(1-32)-OH.

12. [Ala$^{15}$,Leu$^{27}$,Asn$^{28}$]-GRF(1-40)-OH.

13. Eine Verbindung gemäss einem der Ansprüche 1-12 zur veterinärmedizinischen Verwendung, um das Wachstum von Tieren oder ihre Milch-, Fleisch-oder Eierproduktion zu steigern.

14. Eine pharmazeutische Zusammensetzung, die als wirksames Mittel eine Verbindung gemäss einem der Ansprüche 1-12 und ein pharmazeutisch verträgliches, festes oder flüssiges Trägermaterial enthält.

15. Die Verwendung einer Verbindung gemäss einem der Ansprüche 1-12 in der Veterinärmedizin oder zur tierischen Produktion von Milch, Fleisch oder Eiern.